# EUROPEAN PATENT SPECIFICATION

(11) **EP 0 839 913 B1**
(45) Date of publication and mention of the grant of the patent: **21.06.2006**
(21) Application number: 97119273.7
(22) Date of filing: 04.11.1997
(51) Int. Cl.: C12N 15/40, C12N 15/34, C12N 15/31, C12N 15/30, A61K 31/70, C12N 15/88, A61K 38/27

(54) **Method of expressing polypeptides in fish by immersion in DNA-plasmid solution**
Verfahren zur Expression von Polypeptide in Fishen durch wässerung in DNS enthaltende Lösung
Möthode d'expression de polypeptides dans des poissons par immersion dans une solution contenant des plasmides d'ADN .

(30) Priority: 04.11.1996 US 740805; 07.11.1996 EP 96117859
(43) Date of publication of application: 06.05.1998
(73) Proprietor: Ottawa Health Research Institute, Ottawa, Ontario K1Y 4K9 (CA)
(72) Inventor: Davis, Heather L., Ottawa, Ontario, K1S 1T4 (CA)
(74) Representative: Vossius & Partner

(56) References cited:
- EP-A- 0 601 979
- EP-A- 0 773 295
- WO-A-94/29440
- WO-A-96/03034
- FR-A- 2 719 316
- US-A- 5 076 208
- HEPPELL ET AL: "STRAIN VARIABILITY AND LOCALIZATION OF IMPORTANT EPITOPES ON THE MAJOR STRUCTURAL PROTEIN (VP2) OF INFECTIOUS PANCREATIC NECROSIS VIRUS" VIROLOGY, vol. 214, 1995, pages 40-49, XP002084774
- CHU, S. ET AL.: "Structure of the tetragonal surface virulence array protein and gene of Aeromonas salmonicida" JOURNAL OF BIOLOGICAL CHEMISTRY, vol. 266, no. 23, 15 August 1991, pages 15258-65, XP002098110
- CLARK, T.G. ET AL.: "Developmental expression of surface antigen genes in the parasitic ciliate Ichhyophthirius multifiliis." PROC.NATL.ACAD.SCI.USA, vol. 89, July 1992, pages 6363-7, XP002098111
- XIANG Z ET AL: "MANIPULATION OF THE IMMUNE RESP0NSE TO A PLASMID-ENCODED VIRAL ANTIGEN BY COINOCULATION WITH PLASMIDS EXPRESSING CYTOKINES" IMMUNITY, vol. 2, no. 2, February 1995, pages 129-135, XP000670098
- ANDERSON, E.D: ET AL.: "Genetic immunization of rainbow trout (Oncorhynchus mykiss) against infectious hematopoeitic necrosis virus." MOLECULAR MARINE BIOLOGY AND BIOTECHNOLOGY, vol. 5, no. 2, June 1996, pages 114-22, XP002087052
- HANSEN, E. ET AL.: "Strong expression of foreign genes following direct injection into fish muscle." FEBS LETTERS, vol. 290, no. 1-2, September 1991, pages 73-6, XP002087050
- HEPPELL, J. ET AL.: "Expression of foreign genes in fish following direct DNA injection." DEVELOPMENTS IN BIOLOGICAL STANDARDIZATION, vol. 90, January 1997 - March 1997, page 464 XP002087324 & HEPPELL, J. ET AL.: "Expression of foreign genes in fish following direct DNA injection." FISH VACCINOLOGY (MEETING), 5 - 7 June 1996, Oslo, Norway
- NOONAN, B. ET AL.: "Recombinant infectious hematopoietic necrosis virus and viral hemorrhagic septicemia virus glycoprotein epitopes expressed in Aeromonas salmonicida induce protective immunity in rainbow trout (Oncorhynchus mykiss)." APPLIED AND ENVIRONMENTAL MICROBIOLOGY, vol. 61, no. 10, October 1995, pages 3586-91, XP002087051
- DATABASE BIOSIS BIOSCIENCES INFORMATION SERVICE, PHILADELPHIA, PA, US AN 94:179323, DN 97192323, 1993 ESTEPA, A. ET AL.: "Importance of rhabdoviruses in aquaculture. Technological strategies for prevention and control." XP002087053
- KRIEG A M ET AL: "CPG MOTIFS IN BACTERIAL DNA TRIGGER DIRECT B-CELL ACTIVATION" NATURE, vol. 374, 6 April 1995, pages 546-549, XP000197060
- NABEL G J ET AL: "DIRECT GENE TRANSFER FOR IMMUNOTHERAPY AND IMMUNIZATION" TRENDS IN BIOTECHNOLOGY, vol. 11, no. 5, May 1993, pages 211-215, XP000389867
- DAVIS H L ET AL: "PLASMID DNA IS SUPERIOR TO VIRAL VECTORS FOR DIRECT GENE TRANSFER INTO MOUSE SKELETAL MUSCLE" HUMAN GENE THERAPY, vol. 4, 1993, pages 733-740, XP000653531
- COLL J M ET AL: "Applications of monoclonal antibodies in aquaculture" BIOTECHNOLOGY ADVANCES, vol. 13, no. 1, 1995, page 45-73 XP004046558

## Description

Viral and bacterial diseases in fin-fish, shellfish or other aquatic lifeforms pose a serious problem for the aquaculture industry. Owing to the high density of animals in the hatchery tanks or enclosed marine farming areas, infectious diseases may eradicate a large proportion of the stock in, for example, a fin-fish, shellfish, or other aquatic lifeforms facility. Prevention of disease is a more desired remedy to these threats to fish than intervention once the disease is in progress. Vaccination of fish is the only preventative method which may offer long-term protection through immunity.

The fish immune system has many features similar to the mammalian immune system, such as the presence of B cells, T cells, lymphokines, complement, and immunoglobulins. Fish have lymphocyte subclasses with roles that appear similar in many respects to those of the B and T cells of mammals. Additionally, the efficiency of the immune response of fish can be affected by outside stresses, as is true in mammals. However, fish, unlike mammals, display a temperature-dependent development of protective immunity in response to antigens.

Most vaccines for fish have been developed against bacteria while there have been very few fish vaccines made for combating viral or parasitic diseases. Fish have been immunized by antigen-based immunization methods using live attenuated pathogens, killed whole pathogens, or more recently, in laboratory settings, recombinant proteins. While live attenuated vaccines induce good humoral and cell-mediated immune responses and can be administered orally or by immersion or injection, there is the important risk of reversion to a virulent form. Whole live attenuated vaccines are not preferred in industrial farming due to the risk of contaminating other fish - a live attenuated vaccine which may be generally safe for the target species of fish may be virulent in other species of fish.

Fish vaccines using whole killed bacteria (i.e. bacterins) or recombinant proteins from pathogens expressed in cell lines (subunit vaccines) have the disadvantage of inducing short-lived immune responses. Injected antigen, including recombinant protein, is processed solely in an exogenous form usually causing induction of a humoral response (i.e., production of antibodies) but often a failure to induce cell-mediated immunity (i.e., cytotoxic T-cells).

Another disadvantage of whole killed and subunit vaccines is that they almost always must be injected and they require an adjuvant to induce an effective immune response. Intramuscular injections of these adjuvants can cause granuloma formation which scars the flesh and lowers the market value of the fish. Intraperitoneal injection of adjuvants may cause adhesions between the viscera which can affect the health of the fish and retard fish growth.

Recombinant protein vaccines are difficult and expensive to make especially if the protein must be purified. For example, bacterially-expressed recombinant proteins may form inclusion bodies from which recovery of protein in correct configuration may be low or nonexistent. Induction of an immune response may require that the antigenic protein be correctly glycosylated and folded, which may not be accomplished in a cell other than an animal cell.

Some of the current methodologies for administering vaccines are not technically or economically practical. For example, direct injection of recombinant and whole killed pathogen vaccines into the fish is labor intensive and expensive relative to the future market value of the fish. Furthermore; injection needles can cross-infect fish with contaminating pathogenic organisms, and accidental injection of humans can cause severe or fatal infections and anaphylactic reactions. Moreover, noninjurious injection of small fish is very difficult, especially in young fry, which are particularly susceptible to disease.

A less expensive and easier method which has been used to administer killed viral or bacterial vaccines is an oral method wherein the vaccine is added directly to the water or incorporated into fish food. Oral vaccines have historically shown inconsistent and relatively low levels of protection suggesting that they may be best used as a method of revaccination.

Genes have been introduced directly into animals by using live viral vectors containing particular sequences from an adenovirus, an adeno-associated virus, or a retrovirus genome. The viral sequences allow the appropriate processing and packaging of a gene into a virion, which can be introduced to animals through invasive or non-invasive infection. Viral vectors have several disadvantages. Viral vectors being live pathogens, still carry the risk of inadvertent infection. Furthermore, proteins from viral vector sequences induce undesirable inflammatory or other immune responses which may prevent the possibility of using the same vector for a subsequent vaccine or boost. Viral vectors also limit the size of the target gene that can be expressed due to viral packaging constraints.

Naked DNA transfects relatively efficiently if injected into skeletal muscle but poorly or not at all if injected into other tissues (Wolff et al., Science 247:1465-1468 (1990), incorporated herein by reference). Plasmid DNA coated onto the surface of small gold particles and introduced into the skin by a helium-driven particle accelerator or "gene-gun" can directly transfect cells of the epidermis and dermis (Pecorino and Lo, Current Biol., 2:30-32 (1992), which is incorporated herein by reference).

DNA has also been introduced into animal cells by liposome-mediated gene transfer. DNA-liposome complexes, usually containing a mixture of cationic and neutral lipids, are injected into various tissues or instilled into the respiratory passages. Nabel et al., Hum. Gene Ther., 3:649-656 (1992), which is incorporated herein by reference, have shown that liposomes may be used to transfect a wide variety of cell types by intravenous injection in mammals. In addition, liposome-mediated gene transfer has been used to transfer the cystic fibrosis transmembrane conductance gene into the nasal epithelium of mice and humans suffering from cystic fibrosis (Yoshimura et al., Nucleic Acids Reg., 12:3233-3240 (1992) and Caplan et al., Nature Med., 1:39-46 (1995), respectively, both of which are incorporated herein by reference.

Substances may also be administered using biodegradable microspheres composed of polymers such as polyester poly(lactide-co-glycolide) (Marx et al., Science, 260:1323-1328 (1993), incorporated herein by reference). It is notable that these particles can survive the upper digestive system and arrive intact in cells of gut-associated lymphoid tissue (Eldridge et al., Adv. Exp. Med. Biol., 251:191-202 (1989) , incorporated herein by reference). Biodegradable microspheres have been used to deliver recombinant antigens, toxoids or attenuated virus into mammals by systemic and oral routes (O'Hagan et al., Immunology 73:239-242 (1991); O'Hagen et.al., Vaccine 11:149-154 (1993); Eldridge et al., Mol. Immunol, 228:287-293 (1991) incorporated herein by reference). They may also be useful to deliver recombinant plasmid DNA to gut-associated lymphoid tissue for the purpose of immunization.

While most work has been carried out on mammals, plasmid DNA encoding reporter genes have been successfully introduced into fish by intramuscular injection (Hansen et al., FEBS Lett. 290:73-76 (1991), incorporated herein by reference). Thus, cells in fish can express proteins from a foreign gene with the same types of vector constructs (i.e., backbones, promoter and enhancer elements) that are used in mammals.

The induction of an immune response to a protein expressed from an introduced gene was first suggested by Acsadi et al., New Biologist 3:71-81 (1991), which is incorporated herein by reference, who found that after plasmid DNA transfer into rat cardiac muscle, reporter gene expression was transient but could be prolonged by treatment with an immuno-suppressant. Subsequently, it was shown that antibodies were induced in rodents against human growth hormone (Tang et al., Nature, 356:152-154 (1992); Eisenbraun et al., DNA Cell. Biol., 12:791-797 (1993), both of which are incorporated herein by reference) or human α-antitrypsin (Tang et al., Nature, 356:152-154 (1992), also incorporated herein by reference) when these proteins were expressed from DNA coated onto gold particles and introduced into cells of the skin by bombardment.

DNA-based immunization refers to the induction of an immune response to an antigen expressed in vivo from a gene introduced into the animal. This method offers two major advantages over classical vaccination in which some form of the antigen itself is administered. First, the synthesis of antigen in a self-cell mimics in certain respects an infection and thus induces a complete immune response but carries absolutely no risk of infection. Second, foreign gene expression may continue for a sufficient length of time to induce strong and sustained immune responses without boost.

Several mammalian animal models of DNA-based immunization against specific viral, bacterial or parasitic diseases have been reported. These include influenza [(Fynan et al., Proc. Nat'l Acad. Sci. USA, 90:11478-11482 (1993); Montgomery et al., DNA Cell. Biol., 12:777-783 (1993); Robinson et al., Vaccine, 11:957-960(1993); Ulmer et al., Science, 259:1745-1749 (1993)], HIV [Wang et al. (1993)], hepatitis B [Davis et al., Hum. Molec. Genet., 2:1847-1851 (1993)], malaria [Sedagah et al., Proc. Nat'l Acad. Sci., USA, 91:9866-9870 (1994)], bovine herpes [Cox et al., J. Virol, 67:5664-5667 (1993)], herpes simplex (Rousse et al., J. Virol., 68:5685-5689 (1994); Manicken et al. J. Immunol., 155:259-265 (1995)], rabies [Xiang et al., Virology, 199:132-140 (1994)]; lymphocytic chorio-meningitis (Yokoyama et al., J. Virol., 6964:2684-2688 (1995)] and tuberculosis [Lowrie et al., Vaccine, 12:1537-1540 (1994)], all of which are incorporated herein by reference. In most of these studies a full-range of immune responses including antibodies, cytotoxic T lymphocytes (CTL), T-cell help and (where evaluation was possible) protection against challenge was obtained. In these studies naked DNA was introduced by intramuscular or intradermal injection with a needle and syringe or by instillation in the nasal passages, or the naked DNA was coated onto gold particles which were introduced by a particle accelerator into the skin.

There is a need for novel systems to vaccinate fin-fish, shellfish, and other aquatic animals against diseases. These systems should be inexpensive.to produce and administer, avoid the use of live, attenuated organisms, and induce strong and long-lasting immunity preferably without boost and with induction of both antibodies and cell-mediated immunity. More preferably, the system should be applicable to small fish, be less stressful to fish during administration, and have the capacity of simultaneously immunizing many animals for reduced labor-related costs.

The present invention relates to a non-therapeutic method for expressing a polypetide in a fin-fish, by DNA plasmid expression systems by immersing the fin-fish in a DNA plasmid solution. The present invention relates to introduction of DNA plasmids (alone or in a formulation) containing preferably sequences encoding antigenic components of viral, bacterial or parasitic diseases by transfection into fin-fish The DNA sequences according to this invention are present in vectors capable of inducing protein expression of these sequences (i.e. expression vectors) and may be administered alone or in combination with other DNA sequences in the same or other expression vectors or as oligonucleotides. These additional DNA sequences may encode cytokines, costimulatory molecules, or may include immunostimulatory sequences (e.g., CpG motifs). The DNA sequences may also be given with other adjuvants, such as alum.

The present invention also relates to methods of administration of DNA plasmid expression vectors to fin-first which may or may not encode polypeptides from pathogens. DNA vectors of this invention may be administered to aquaculture species by immersion.

Fin-fish include all vertebrate fish, which may be bony or cartilaginous fish. A preferred embodiment of this invention is the immunization of fin-fish. These fin-fish include but are not limited to salmonids, carp, catfish, yellowtail, seabream, and seabass. Salmonids are a family of fin-fish which include trout (including rainbow trout), salmon, and Arctic char.

Purification of DNA on a large scale may be accomplished by anion exchange chromatography (for example, resins manufactured by Qiagen, U.S. FDA Drug Master File (DMF-6224)).

DNA which is introduced to fin-fish will encode foreign polypeptides (e.g., those derived from viral, bacterial or parasitic pathogens). Polypeptides of this invention refer to complete proteins or fragments thereof, including peptides which are epitopes (e.g., a CTL epitope) associated with an infectious virus, bacterium or parasite.

In preferred embodiments, the DNA sequences encoding polypeptides of viral pathogens may be selected from the group consisting of glycoprotein (G) or nucleoprotein (N) of viral hemorrhagic septicemia virus (VHSV); G or N proteins of infectious hematopoietic necrosis virus (IHNV); VP1, VP2, VP3 or N structural proteins of infectious pancreatic necrosis virus (IPNV); G protein of spring viremia of carp (SVC); and a membrane-associated protein, tegumin or capsid protein or glycoprotein of channel catfish virus (CCV).

In other preferred embodiments, the DNA sequences encoding polypeptides of bacterial pathogens may be selected from the group consisting of an iron-regulated outer membrane protein, (IROMP), an outer membrane protein (OMP), and an A-protein of *Aeromonis salmonicida* which causes furunculosis, p57 protein of *Renibacterium salmoninarum* which causes bacterial kidney disease (BKD), major surface associated antigen (msa), a surface expressed cytotoxin (mpr), a surface expressed hemolysin (ish), and a flagellar antigen of *Yersiniosis;* an extracellular protein (ECP), an iron-regulated outer membrane protein (IROMP), and a structural protein of *Pasteurellosis;* an OMP and a flagellar protein of *Vibrosis anguillarum and V. ordalii:* a flagellar protein, an OMP protein, aroA, and purA of *Edwardsiellosis ictaluri* and E. *tarda*; and surface antigen of *Ichthyophthirius;* and a structural and regulatory protein of *Cytophaga columnari*; and a structural and regulatory protein of *Rickettsia*.

In yet another preferred embodiment, the DNA sequences encoding polypeptides of a parasitic pathogen may be selected from one of the surface antigens of *Ichthyophthirius.*

The methods of this invention could also be used to introduce plasmid vectors encoding polypeptides endogenous to the animal, but which might be normally present in low concentrations (e.g., growth hormones). In this case the expression proteins would serve a physiological role (i.e. enhanced growth) rather than induce an immune response.

Vectors useful in the making of expression plasmids include, but are not limited to, vectors containing constitutive promoters, inducible promoters, tissue-specific promoters, or promoters from the gene of the antigen being expressed. Constitutive promoters may include strong viral promoters, for example, promoter sequences from cytomegalovirus (CMV), Rous sarcoma virus (RSV), simian virus-40 (SV40), or herpes simplex virus (HSV). Tissue-specific promoters may include the muscle beta-actin promoter or the thymidine kinase promoter. An inducible or regulatable promoter, for example, may include a growth hormone regulatable promoter, a promoter under the control of *lac* operon sequences or an antibiotic inducible promoter or a Zinc-inducible metallothionein promoter.

The vector should include an expression control sequence comprising a promoter (e.g., inducible or constitutive promoters described above) DNA sequence, and may include, but is not limited to, an enhancer element, an RNA processing sequence such as an intronic sequence for splicing of a transcript or a polyadenylation signal (e.g., from simian virus-40 (SV40) or bovine growth hormone (BGH)), a signal sequence for secretion of the expressed protein, or one or more copies of immunostimulatory DNA sequences known as CpG motifs. The vector should also include one or more of the following DNA sequences: bacterial origin of replication sequences, a selectable marker, which may be for antibiotic resistance (e.g., kanamycin) or for non-antibiotic resistance (e.g., β-galactosidase gene).

An additional preferred embodiment of this invention relates to the administration of a vector containing one or more different DNA sequences, one sequence encoding an antigen and the others encoding polypeptides which may or may not be antigenic. For example, the vector may encode two antigens from the same pathogen. Alternatively, the different antigen(s) may the from different pathogen.

When two or more polypeptide-encoding DNA sequences are present in one vector, the transcription of each antigen-encoding DNA sequence may be directed from its own promoter. Alternatively, one promoter may drive the expression of two or more antigen-encoding DNA sequences joined in frame to each other to express a fusion protein. For example, VP2 and VP3 proteins of infectious pancreatic necrosis virus (IPNV) may be fused. In another embodiment, DNA sequences encoding two or more antigens from different diseases may be joined.

Alternatively, a DNA sequence encoding an antigenic polypeptide may be fused to a DNA sequence encoding a carrier polypeptide. In a preferred embodiment, the carrier polypeptide may contain one or more envelope proteins of the hepatitis B virus, preferably from the human hepatitis B virus. In a more preferred embodiment, the envelope proteins of hepatitis B virus will be the small and major protein (also referred to as surface antigen).

In another embodiment, each polypeptide-encoding DNA sequence in the vector may be under the control of its own promoter for expression of two or more .non-fused polypeptides.

Alternatively, the DNA sequences encoding additional antigens may be administered by using a second vector containing such sequences. Such sequences may encode antigens from the same pathogen or different pathogens, or cytokines, cholera toxin subunits, or other immunostimulants. Such a vector may be administered concurrently or sequentially with the first expression vector. A preferred embodiment of this invention is the concurrent administration of expression vectors. One vector may be induced to express protein simultaneously with or after expression of protein from the other vector.

In yet another embodiment of this invention, antigen-expressing vectors may be administered concurrently with an antigen-based vaccine such as a recombinant protein or whole-killed vaccine. In a preferred embodiment, the antigen-expressing vector is administered simultaneously with a protein antigen (i.e. recombinant protein or whole killed pathogen) .

The DNA used in the method of this invention is preferably purified plasmid DNA(s) simply dissolved in an aqueous solution or in a formulation. One of skill in the art would readily appreciate how to formulate DNA used in the methods of this invention with known transfection reagents such as cationic liposomes, fluorocarbon emulsions, cochleates, tubules, gold particles, biodegradable microspheres, or cationic polymers.

Liposomes useful for transfection of DNA of this invention include commercially available liposomes and liposomes containing either cationic lipids or cationic polymers. In a preferred embodiment of this invention, liposomes would include a mixture of a neutral lipid such as dioleoylphosphatidylethanolamine (DOPE) or cholesterol and a cationic lipid.

In a more preferred aspect of the invention, liposomes would include a mixture of cationic polymers and neutral lipids such as DOPE or cholesterol. Such liposomes may be prepared as described herein and in United States Provisional Patent Application entitled, "A Novel Class of Cationic Reagents for High Efficient and Cell-Type-Specific Introduction of Nucleic Acids into Eukaryotic Cells", incorporated by reference herein. Unlike cationic lipids, cationic polymers do not have ester-linkages and have greater stability in vivo as a result. Cationic polymers (also referred to as dendrimers) may be dimeric, cyclic, oligomeric, or polymeric in structure.

Cationic polymers in an aqueous solution without neutral lipids are also preferred transfection reagents according to the preferred embodiments of this invention. Cationic polymers have been shown to work well for transfecting fish cells in vitro with plasmids expressing fish pathogen antigens (see Table 1, Example 1).

Cochleates, which are stable phospholipid-calcium precipitates composed of phosphatidylserine, cholesterol and calcium are desirable non-toxic and non-inflammatory transfection reagents that can survive the digestive system. Biodegradable microspheres composed of polymers such as polyester poly(lactide-co-glycolide) have been used to microencapsulate DNA for transfection.

Tubules have been previously described in the literature as lipid-based microcylinders consisting of helically wrapped bilayers of lipid, the edges of which are packed together. DNA may be placed in the hollow center for delivery and controlled release in animals.

With immersion, DNA may enter cells of the epithelium of the skin, the gills or the gut wall. DNA may then be expressed in these transfected cells leading to induction of appropriate immune responses in regional or systemic lymphoid tissue.

In a more preferred embodiment of this invention, a large number of animals can be transfected simultaneously by immersion in a solution containing DNA. In one embodiment, fish are dip-netted into suspensions containing DNA formulations (e.g., DNA formulated with cationic polymers or liposomes) for at least several seconds. The fish are then returned to the holding tanks in which they develop immunity. In another embodiment, fin-fish, are placed into tanks containing a relatively small volume of water. Concentrated DNA formulations (e.g., DNA formulated with cationic polymers or liposomes) is added to the tank, and animals are left for a period of time up to several hours before the tank is refilled with water to restore the normal aquatic environment.

The following examples illustrate the invention

### Examples

### Example 1 - Cloning of DNA encoding Antigenic Proteins Into Plasmid DNA Vectors

DNA encoding proteins of fish pathogens is useful in developing DNA fish vaccines. Table 1 below recites fish pathogen protein expression plasmids. Table 1 describes nucleotide sequences encoding proteins from pathogens cloned into vectors having the cytomegalovirus promoter (CMV), i.e., pcDNA3 (from Invitrogen) or a vector containing the CMV promoter and intron A of CMV to promote better expression of protein (pCMV_{A} vector). For example, genetic sequences coding for the major glycoprotein (G) or nucleoprotein (N) of the viral hemorrhagic septicemia virus (VHSV) were cloned into the EcoRI site of either the pcDNA3 or pCMV_{A} vector. Nucleotide sequences encoding the VP2 and VP3 structural proteins of the infectious pancreatic necrosis virus (IPNV) were cloned into same vectors. The gene encoding the ferric siderophore receptor (fstA) of Aeromonas *salmonicida* has also been cloned into expression vectors. The fstA protein is one of several possible iron-regulated outer membrane proteins that could be expressed as an antigen from a DNA vaccine.

**Table 1**

| Plasmid | Vector | Antigen | Pathogen |
|---|---|---|---|
| pCMV-G | pcDNA3 | G glycoprotein | viral hemorrhagic |
| | (EcoRI site) | (#1-1565)* | septicemia virus |
| pCMV_{A}-G | pCMV_{A} vector | G glycoprotein | viral hemorrhagic |
| | (EcoRI site) | (#1-1565)* | septicemia virus |
| pCMV-N | pcDNA3 | N nucleoprotein | viral hemorrhagic |
| | (EcoRI site) | (#92-1306)* | septicemia virus |
| pCMV_{A}-N | pCMV_{A} vector | N nucleoprotein | viral hemorrhagic |
| | (EcoRI site) | (#92-1306)* | septicemia virus |
| pCMV-VP2 | pcDNA3 | VP2 | infectious pancreatic |
| | (HindIII/XbaI site) | (#117-1760)* | necrosis virus |
| pCW_{A}-VP2 | pCMV_{A} vector | VP2 | infectious pancreatic necrosis virus |
| | (Sall/XbaI) | (#117-1760) * | |
| pCMV-VP3 | pcDNA3 | VP3 | infectious pancreatic |
| | (EcoRI/XbaI site) | (#2325-3011)* | necrosis virus |
| pCMV_{A}-VP3 | pCMV_{A} vector | VP3 | infectious pancreatic |
| | (EcoRI/XbaI site) | (#2325-3011)* | necrosis virus |
| pCMV-fstA | pcDNA3 | IROMP fstA | *Aeromonis salmonicida* |
| | (EcoRI/XbaI site) | (#76-2630)* | |
| pCMV_{A}- fstA | pCMV_{A} vector | IROMP fstA | *Aeromonis salmonicida* |
| | (EcoRI/Xbal site) | (#76-2630)* | |

| | | | |
|---|---|---|---|
| * # indicates the nucleotide sequences within the genome of the pathogen which have been cloned to code for the antigen | | | |

### Reference Example 1 - Expression of Foreign Protein In Fish Injected with Pure Plasmid DNA Vector

The pCMV-luc plasmid used in the following experiments contains the luciferase reporter gene (luc) under the control of the cytomegalovirus promoter.
Purified plasmid DNA was prepared by using commercially available Qiagen DNA purification columns. The purified plasmid DNA was then dissolved in endotoxin-free Dulbecco's phosphate buffered saline (DPBS) without calcium chloride or magnesium chloride, or in 0.15 M NaCl dissolved in deionized distilled water for a final concentration of 0.001 mg/ml to 5 mg/ml DNA. Fish were anaesthetized with 0.168 mg/ml tricaine (3-amino benzoic acid ethylester) in water or by placing the fish on ice for 30-60 seconds before injection. Trout and zebra fish were injected intramuscularly between the dorsal fin and the lateral line with 10 *µ*l of the DNA solution.

Luciferase activity was measured in the muscle and gills 2.5 days after injection. Rainbow trout were euthanized by an overdose of tricaine (0.1% w/v). Zebra fish were killed by immersion in ice. The muscle or gills of the fish were removed on ice, homogenized, centrifuged to pellet cellular debris, and the supernatants containing soluble proteins were assayed for luciferase activity. Luciferase assays were carried out using a kit commercially available from Promega Corporation. Light emission in relative light units (RLU) was quantitated by a luminometer (Analytical Luminescence Laboratory) over a ten second interval and background values from control samples were subtracted. The concentration of protein in the supernatants was determined and luciferase activity was expressed as RLU/sec/mg protein.

The results summarized in Table 2 (below) indicate that purified plasmid DNA can efficiently transfect fish cells after intramuscular injection. Nanogram amounts of plasmid DNA were able to induce detectable protein expression in both the injected muscle as well as more distant cells (e.g., in gills), showing that different types of cells, possibly including antigen presenting cells (APC), are transfectable by plasmid DNA and are capable of synthesis of foreign protein. Cells distant to the site of injection (i.e. gills) expressed lower amounts of protein than the injected muscle cells. Zebra fish and trout are not closely related species of fish. Therefore, the results in Table 2 indicate that most species of fish could take up and express foreign proteins from injected plasmids.

**Table 2**

| Dose of DNA (µg) | Luciferase Activity (RLU/sec/mg of protein)* | | | |
|---|---|---|---|---|
| | Trout | | Zebra Fish | |
| | Muscle | Gills | Muscle | Gills |
| 0.01 | 3,449 | 4 | 502 | 18 |
| | (±1548) | (±3) | (±307) | (±8) |
| 0.1 | 22,768 | 36 | 11,665 | 94 |
| | (±12,708) | (±14) | (±2.989) | (±66) |
| 1 | 78,408 | 618 | 826,486 | 228 |
| | (±51,523) | (±567) | (±368,790) | (±115) |
| 10 | 280,051 | 982 | 199,285 | 833 |
| | (±172,749) | (±743) | (±97,134) | (±621) |
| 50 | 417,226 | 980 | 145,891 | 5,519 |
| | (±165.164) | (±393) | (±85.645) | (±4791) |

| | | | | |
|---|---|---|---|---|
| *mean ± standard error of mean (n=10 fish per group) | | | | |

### Example 2 -- Expression of Plasmid DNA after Immersion of Fish in DNA-containing Solutions

Cationic lipid, G304, was obtained from Gibco BRL, New York, USA. Cationic polymer liposomes designated Q203, Q205, Q206, Q208, Q250, and QX were obtained from Qiagen GmbH, Hilden, Germany. Cationic polymer liposomes are composed of a mixture of cationic polymers and neutral lipids. Such transfection reagents were prepared as described in U.S. Provisional Patent Application entitled, "A Novel Class of Cationic Reagents for High Efficient and Cell-Type-Specific Introduction of Nucleic Acids into Eukaryotic Cells", incorporated by reference herein.

For example, a cationic polymer (either Q203, Q205, Q206, Q208, Q250 or QX, described below in Table 3) and a neutral lipid, dioleoyloxiphosphatidylethanolamine (DOPE) were mixed together for a final concentration of 2 mM in chloroform, which was then evaporated off in a rotary evaporator at 60°C. The mixture was dried for 10 minutes under a reduced pressure of 10 to 15 mbar. Under sterile conditions, endotoxin free deionized water was added to the mixture, which was then heated while stirring at 60°C.

Next, Q203, Q205, Q250, and QX were sonicated once for 300 seconds at 60°C. In the case of Q250, trans β carotene was added to a final concentration of 0.37 mM before sonication. Q206 and Q208 were not sonicated but were stirred at 60°C until the solutions became transparent or slightly opalescent. The total concentration of DOPE+cationic polymer for all liposomes was 2 mM. The concentration of DOPE in each liposome can be calculated by multiplying the X(DOPE) value in Table 3 by 2 mM so that, for example, Q203-containing liposomes are 1.7 mM DOPE and 0.3 mM Q203. Table 3 (below) summarizes the cationic polymer liposomes used in the methods of this invention.

**Table 3**

| Cationic Polymer Liposome Reagent | Cationic Polymers | X(DOPE) | Method of Preparation |
|---|---|---|---|
| Q203 | butandiyl-1,4-bis(octadecyl dimethylammonium bromide) | 0.85 | with sonication. |
| Q205 | butandiyl-1,4-bis(octadecyl dimethylammonium bromide) | 0.82 | with sonication. |
| Q206 | butandiyl- 1,4-bis(octadecyl dimethylammonium bromide) | 0.78 | without sonication. |
| Q208 | butandiyl-1,4-bis(octadecyl dimethylammonium bromide) | 0.75 | without sonication. |
| Q250 | didodecyldimethyl ammonium bromide | 0.571 | with sonication. Add trans β carotene to final concentration of 0.37 mM. |
| QX | didodecyldimethyl ammonium bromide | 0.375 | with sonication. with sonication. |

DNA:liposome complexes were prepared by independently diluting DNA and liposome solutions in 0.15 M NaCl, then mixing the two solutions and vortexing, and then incubating the mixture at room temperature for 30-45 minutes. The solutions were diluted further with water and incubated for an additional 10-15 minutes at room temperature prior to use with fish.

Each fish was immersed in the solution of liposome formulated DNA (2.5 ml or 5 ml per fish) for 90 minutes and then returned to its normal holding tank. After 2.5 days, the fish were homogenized or gills and muscle were homogenized separately and assayed for luciferase activity.

Table 4 (below) shows luciferase activity above background in individual zebra fish after immersion. Thus, the results of Table 8 indicate that the majority of fish were successfully transfected and able to express foreign protein after immersion in DNA:liposome solutions. No fish expressed luciferase activity after immersion in pCMV-luc DNA without liposomes. Therefore, lipid-containing transfection reagents appear to significantly contribute to the transfection efficiency of DNA into fish with the immersion technique.

**Table 4**

| Transfection Reagent | Total Luciferase Activity (RLU/ second) |
|---|---|
| G304 | 330, 65, 0, 1643, 1581, 143, 5, 165, 0, 0, 257 |
| Q203 | 268, 82, 106, 254 |
| Q205 | 188, 268, 166, 136 |
| Q206 | 208, 286, 170, 108, 174 |
| Q208 | 668, 204, 1060, 0, 0, 180, 842, 242, 90, 36 |
| Q250 | 358, 398, 60, 10, 134, 1742, 54, 136, 84, 136 |
| QX | 166, 80, 302, 74, 432, 630, 28, 28, 260, 260 |

## Claims

1. A non-therapeutic method for expressing a polypeptide in a fin-fish comprising immersing the fin-fish in a DNA-plasmid solution encoding the polypeptide.

2. The method of claim 1, wherein the polypeptide is an antigenic component of a viral, bacterial or parasitic disease.

3. The method according to claim 1 or 2, wherein the polypeptide is selected from at least one of the group consisting of glycoprotein (G) or nucleoprotein (N) of viral hemorrhagic septicemia virus (VHSV); G or N proteins of infectious hematopoietic necrosis virus (IHNV); VP1, VP2, VP3 or N structural proteins of infectious pancreatic necrosis virus (IPNV); G protein of spring viremia of carp (SVC); or a membrane-associated protein, tegumin, a capsid protein, or a glycoprotein of channel catfish virus (CCV).

4. The method according to claim 1 or 2, wherein the polypeptide is selected from at least one of the group consisting of an iron-regulated outer membrane protein (IROMP), an outer membrane protein (OMP), or an A-protein of *Aeromonis salmonicida;* a p57 protein of *Renibacterium salmoninarum;* a major surface associated antigen (msa), a surface expressed cytotoxin (mpr), a surface expressed hemolysin (ish), or a flagellar antigen of *Yersiniosis;* an extracellular protein (ECP), an iron-regulated outer membrane protein (IROMP), or a structural protein of *Pasteurellosis;* an OMP or a flagellar protein of *Vibrosis anguillarum or V. ordalii;* a flagellar protein, an OMP protein, aroA, or purA of *Edwardsiellosis ictaluri or E. tarda;* or a structural or regulatory protein of *Cytophaga columnari;* or a structural or regulatory protein of *Rickettsia.*

5. The method according to claim 1 or 2, wherein the polypeptide is a surface antigen of *lchthyophthirius.*

6. The method according to claim 1 or 2, wherein the polypeptide-encoding DNA sequence encodes a growth hormone.

7. The method according to anyone of claims 3 to 5, wherein the polypeptide-encoding DNA sequence encodes a first and a second polypeptide from the same pathogen.

8. The method according to anyone of claims 1 to 5 or 7, wherein the polypeptide-encoding. DNA sequence additionally encodes a polypeptide from a different pathogen.

9. The method according to anyone of claims 1 to 8, wherein the polypeptide-encoding DNA sequence additionally encodes a carrier polypeptide to form a fusion protein with the polypeptide.

10. The method according to anyone of claims 1 to 9. wherein the polypeptide-encoding DNA sequence additionally encodes a second polypeptide.

11. The method according to claim 10, wherein the second polypeptide is a cytokine.

12. The method according to anyone of claims 1 to 11, wherein the DNA plasmid is formulated with a cationic polymer or a liposome.

13. The method according to claim 12, wherein the liposome contains DOPE.

## Patentansprüche

1. Nicht-therapeutisches Verfahren zum Exprimieren eines Polypeptids in einem Flossenfisch, umfassend das Eintauchen des Flossenfisches in eine Lösung eines DNA-Plasmids, das das Polypeptid codiert.

2. Verfahren nach Anspruch 1, wobei das Polypeptid eine antigenische Komponente einer viralen, bakteriellen oder parasitären Krankheit ist.

3. Verfahren nach Anspruch 1 oder 2, wobei das Polypeptid ausgewählt ist aus mindestens einem aus der Gruppe bestehend aus Glycoprotein (G) oder Nucleoprotein (N) des Virus der viralen hämorrhagischen Septikämie (VHSV); G- oder N-Proteinen des Virus der infektiösen hämatopoietischen Nekrose (IHNV); den strukturellen Proteinen VP1, VP2, VP3 oder N des Virus der infektiösen Pankreasnekrose (IPNV); dem G-Prötein der Frühlingsvirämie des Karpfen (SVC); oder einem Membran-assoziierten Protein, Tegumin, einem Kapsidprotein oder einem Glycoprotein des Channel Catfish-Virus (CCV).

4. Verfahren nach Anspruch 1 oder 2, wobei das Polypeptid ausgewählt ist aus zumindest einem aus der Gruppe bestehend aus einem eisenregulierten äußeren Membranprotein (IROMP), einem äußeren Membranprotein (OMP), oder einem A-Protein von *Aeromonis salmonicida;* einem p57-Protein von *Renibacterium salmoninarum;* einem Haupt-oberflächenassoziierten Antigen (msa), einem Oberflächen-exprimierten Cytotoxin (mpr), einem Oberflächen-exprimierten Hämolysin (ish) oder einem flagellaren Antigen von *Yersiniosis;* einem extrazellulären Protein (ECP), einem eisenregulierten äußeren Membranprotein (IROMP), oder einem strukturellen Protein von *Pasteurellosis;* einem OMP oder einem flagellaren Protein von *Vibrosis anguillarum* oder *V. ordalii;* einem flagellaren Protein, einem OMP-Protein, aroA oder purA von *Edwardsiellosis ictaluri* oder *E. tarda;* oder einem strukturellen oder regulatorischen Protein von *Cytophaga columnari;* oder einem strukturellen oder regulatorischen Protein von *Rickettsia.*

5. Verfahren nach Anspruch 1 oder 2, wobei das Polypeptid ein Oberflächenantigen von *Ichthyophthirius* ist.

6. Verfahren nach Anspruch 1 oder 2, wobei die Polypeptid-codierende DNA-Sequenz ein Wachstumshormon codiert.

7. Verfahren nach einem der Ansprüche 3 bis 5, wobei die Polypeptid-codierende DNA-Sequenz ein erstes und ein zweites Polypeptid codiert, die vom gleichen Pathogen stammen.

8. Verfahren nach einem der Ansprüche 1 bis 5 oder 7, wobei die Polypeptid-codierende DNA-Sequenz zusätzlich ein Polypeptid von einem unterschiedlichen Pathogen codiert.

9. Verfahren nach einem der Ansprüche 1 bis 8, wobei die Polypeptid-codierende DNA-Sequenz zusätzlich ein Trägerpolypeptid codiert, um ein Fusionsprotein mit dem Polypeptid zu bilden.

10. Verfahren nach einem der Ansprüche 1 bis 9, wobei die Polypeptid-codierende DNA-Sequenz zusätzlich ein zweites Polypeptid codiert.

11. Verfahren nach Anspruch 10, wobei das zweite Polypeptid ein Cytokin ist.

12. Verfahren nach einem der Ansprüche 1 bis 11, wobei das DNA-Plasmid mit einem kationischen Polymer oder einem Liposom formuliert ist.

13. Verfahren nach Anspruch 12, wobei das Liposom DOPE enthält.

## Revendications

1. Méthode non-thérapeutique pour exprimer un polypeptide dans un poisson à nageoires comprenant l'immersion du poisson à nageoires dans une solution contenant un plasmide d'ADN codant pour le polypeptide.

2. Méthode selon la revendication 1, dans laquelle le polypeptide est un composant antigénique d'une maladie virale, bactérienne ou parasitaire.

3. Méthode selon la revendication 1 ou 2, dans laquelle le polypeptide est sélectionné dans au moins un du groupe consistant en une glycoprotéine (G) ou une nucléoprotéine (N) du virus de la septicémie hémorragique virale (VHSV) ; des protéines G ou N du virus de la nécrose hématopoïétique infectieuse (IHNV) ; VP1, VP2, VP3 ou des protéines structurales N du virus de la nécrose pancréatique infectieuse (IPNV) ; une protéine G de la virémie printanière de la carpe (SVC) ; ou une protéine associée à la membrane, une tégumine, une protéine de capside, ou une glycoprotéine du virus du poisson chat (CCV).

4. Méthode selon la revendication 1 ou 2, dans laquelle le polypeptide est sélectionné dans au moins un du groupe consistant en une protéine de la membrane externe régulée par le fer (IROMP), une protéine de la membrane externe (OMP), ou une protéine A *d'Aeromonis salmonicida ;* une protéine p57 de *Renibacterium salmoninarum ;* un antigène majeur associé à la surface (msa), une cytotoxine exprimée à la surface (mpr), une hémolysine exprimée à la surface (ish), ou un antigène flagellaire de *Yersiniosis ;* une protéine extracellulaire (ECP), une protéine de la membrane externe régulée par le fer (IROMP), ou une protéine structurale de *Pasteurellosis ;* une OMP ou une protéine flagellaire de *Vibrosis anguillarum* ou *V. ordalii ;* une protéine flagellaire, une protéine OMP, aroA ou purA de *Edwardsiellosis ictaluri* ou E. *tarda ;* ou une protéine structurale ou de régulation de *Cytophaga columnari ;* ou une protéine structural ou de régulation de *Rickettsia.*

5. Méthode selon la revendication 1 ou 2, dans laquelle le polypeptide est un antigène de surface de *Ichthyophthirius.*

6. Méthode selon la revendication 1 ou 2, dans laquelle la séquence d'ADN codant pour le polypeptide code une hormone de croissance.

7. Méthode selon l'une quelconque des revendications 3 à 5, dans laquelle la séquence d'ADN codant pour le polypeptide code un premier et un second polypeptides du même pathogène.

8. Méthode selon l'une quelconque des revendications 1 à 5 ou 7, dans laquelle la séquence d'ADN codant pour le polypeptide code en outre un polypeptide d'un pathogène différent.

9. Méthode selon l'une quelconque des revendications 1 à 8, dans laquelle la séquence d'ADN codant pour le polypeptide code en outre un polypeptide porteur pour former une protéine de fusion avec le polypeptide.

10. Méthode selon l'une quelconque des revendications 1 à 9, dans laquelle la séquence d'ADN codant pour le polypeptide code en outre un second polypeptide.

11. Méthode selon la revendication 10, dans laquelle le second polypeptide est une cytokine.

12. Méthode selon l'une quelconque des revendications 1 à 11, dans laquelle le plasmide d'ADN est formulé avec un polymère cationique ou un liposome.

13. Méthode selon la revendication 12, dans laquelle le liposome contient du DOPE.
